# EUROPEAN PATENT APPLICATION

(11) **EP 1 568 394 A1**
(43) Date of publication of application: **31.08.2005**
(21) Application number: 04075629.8
(22) Date of filing: 26.02.2004
(51) Int. Cl.: A61N 1/05

(54) **Coated electrode**

(71) Applicant: Muijs van de Moer, Wouter Matthijs, 3055 VB Rotterdam (NL)
(72) Inventor: Muijs van de Moer, Wouter Matthijs, 3055 VB Rotterdam (NL)
(74) Representative: Prins, Adrianus Willem

(57) **Abstract**

The invention relates to an anchor electrode for a pacemaker lead. The invention further relates to a pacemaker lead comprising said electrode. In accordance with the invention, the electrode has an anchoring surface which is at least partially covered with an immunosuppressive or cytotoxic agent.

## Description

The invention relates to an electrode provided with a coating comprising a biological active compound, in particular an electrode for a cardiac pacemaker. The invention further relates to a specific medical use of said coating.

In situations of certain disorders of the heart, in particular heart dysrhythmias, it has become widespread practice to stimulate the heart muscles by applying an electrical impulse. Dysrhythmias cause problems because the heart is unable to pump an adequate amount of blood to the body. If the heart rate is too slow, the blood is pumped too slowly. If the heart rate is too fast or too irregular, the heart chambers are unable to fill up with enough blood to pump out with each beat. When the body does not receive enough blood, symptoms such as fatigue, dizziness, fainting, and/or chest pain may occur. These symptoms may be treated with medication or procedures such as a cardiac ablation (removal of a location in the heart that is causing a dysrhythmia by freezing or radiofrequency). If the his-bundle ablated a pacemaker is inserted (to signal the heart to beat when the heartbeat is too slow), or an implantable cardioverter defibrillator (to sense when the heart is beating too fast and to deliver an electrical shock to slow the heart down).

A pacemaker is a small electronic device composed of three parts: a generator, at least one lead, and an electrode provided on a tip portion of the lead. The generator is the "brain" of the device. It is a small metal case that contains electronic circuitry and a battery. The lead is an insulated wire that is connected to the generator on one end, with the electrode at the tip portion on the end. The electrode is placed inside one of the heart's chambers. The electrode on the lead tip touches the heart wall and senses the heart's electrical activity. This information is relayed to the generator by the lead. If the heart's rate is slower than the low limit programmed into the generator, an electrical impulse is sent through the lead to the electrode and the pacemaker's electrical impulse causes the heart to beat at a faster rate. When the heart is beating at a rate faster than the programmed low limit, the pacemaker will monitor the heart rate. No electrical impulses will be sent to the heart unless the heart's natural rate falls below the pacemaker's low limit.

An internal cardioverter defibrillator (ICD) looks very similar to a pacemaker: it has a generator, at least one lead, and an electrode provided on a tip portion of the lead. These components work very much like a pacemaker. However, the ICD is designed to deliver an electrical shock to the heart when the heart rate becomes dangerously fast. Newer ICDs can also be programmed to function as pacemakers in addition to the cardioversion and defibrillation functions. For the sake of simplicity, herein below the term pacemaker will be used to refer to both a pacemaker and an ICD.

The procedure for inserting a pacemaker typically involves making a small incision just under the collarbone. The pacemaker generator will be attached to the lead and then inserted into the small pocket made for it under the collarbone. The pacemaker lead is inserted into the heart through a vein, usually through the subclavian vein. Typically, the lead is inserted through the left subclavian vein into the right atrium and right ventricle of the heart. The incision is closed, and the procedure is finished.

Under certain circumstances, it may be desired to use a pacemaker to stimulate the heart from the left side. Sometimes the left side of the heart will be stimulated in addition to the right side; sometimes it will be stimulated instead of the right side.

Research has shown that it is beneficial to stimulate the right ventricle in balance with a stimulation of the left ventricle. The cardiac output can be improved in the case of a failing heart by stimulating both ventricles (chambers) by the pacemaker. Even a small time difference in pacing between the left and right ventricles may show a measurable improvement of the cardiac output. This is different per patient. The time interval between the stimulation spikes to the right and left ventricle has to be measured during implantation of the pacemaker in coherence to the cardiac output per patient. What is the optimum mode depends on a complexity of factors in each case.

In order to stimulate the left side of the heart, a pacemaker lead may be inserted into the heart through the coronary vein (vena coronaria), analogous to the insertion of a lead into the right side of the heart through the subclavian vein. However, in contrast to the atrium and right ventricle, the coronary vein has a smooth inner wall without trabeculae, which makes a proper and stable positioning of the electrode at the tip of the lead difficult, if not impossible.

An alternative could be to position the lead within the artery by making use of a wedge-like configuration. However, the coronary artery is of too small a diameter to allow this configuration to work without closing off the blood flow through the artery completely.

The use of leads provided with an anchor electrode at the tip e.g. a screw type electrode, to fix the electrode in the wall of the left atrium and/or ventricle has also been proposed. However, proper fixation of the tip into the left atrium and/or ventricle through the subclavian artery causes damage to the aortic valve. Also it is shown to give rise to a dysrhythmia, which is the condition that the pacemaker is intended to treat.

For these reasons, pacemakers have only rarely been used to stimulate the left side of the heart. When done, this involved the use of leads having anchor electrodes which were attached in the myocard, at the outside of the left atrium and/or ventricle of the heart. This has been done only occasionally as it requires opening of the thorax cavity.

The use of pacemakers to stimulate the left side of the heart has, however, also not been widely adopted as the anchor electrodes that are used are associated with yet another serious disadvantage.

The fixation of the electrode on the lead tip of the pacemaker by anchoring it into the myocard causes damage to the tissue of the myocard. Although the tissue will normally fully recover from this damage, the healing process will generally give rise to the formation of scar tissue. The scar tissue, which is the result of excessive growth of fibroblasts at the site of the wound caused by the attachment of the lead tip, forms a barrier for the electrical current to reach the myocardial cells which require stimulation. Thus, the electrical resistance is increased and a larger current is required to provide the same electrical stimulus. As a result, the pacemaker's batteries run out sooner requiring sooner replacement or recharging.

The present invention aims to overcome these problems, as well as other problems which will become apparent from the detailed discussion below.

In accordance with the invention, it has been found that formation of scar tissue at the site of an anchor electrode of a lead tip of a pacemaker being anchored into the myocard can be significantly reduced, or even substantially prevented, by making use of an anchor electrode that is provided with a specific coating. Reduction or prevention of scar tissue formation has the advantage that the electrical resistance encountered by the electrical current flowing from the pacemaker to the heart muscle tissue is better controlled and the operational time of the batteries of the pacemaker is optimized.

Accordingly, the invention relates to an anchor electrode for a pacemaker lead electrode having an anchoring surface that is at least partially coated with a coating comprising an immunosuppresive or cytostatic agent.

It has been found that a coating on an anchor electrode for a pacemaker lead according to the invention significantly inhibits excessive proliferation of fibroblasts, and thus reduces or even prevents formation of scar tissue, despite the damage the lead tip does to the tissue of the myocard by its attachment through the anchoring feature. As a result, there will be less or no increase of the electrical resistance encountered by the electrical impulse the pacemaker provides to the tissue of the myocard and the batteries of the pacemaker will have a considerably longer running time.

Recent developments in minimal invasive surgical techniques have made it possible to insert a lead of a pacemaker, e.g. by making use of an endoscope and to attach the coated anchor electrode of the lead directly the myocard on the left side of the heart, e.g. by screwing or stapling. This way, stimulating of the left side of the heart can be carried out in a simple yet effective manor.

As mentioned above, the anchor electrode is according to the invention provided with an anchoring surface, e.g. the surface of the electrode that cooperates with the myocard, that is at least partially coated with a coating comprising an immunosuppressive or cytostatic agent.

The anchor electrode may e.g. be provided with barbs, a beard or a fluke to cooperate with the myocard. Preferably, the anchor electrode comprises a screw portion, e.g. a helically wound wire or a threaded portion.

The anchor electrode may comprise a contacting plate portion and a fastener portion, the fastener portion being at least partially covered with the coating. The fastener may e.g. be formed as a screw or staple.

Preferably, the anchor electrode comprises a segmented electrical contact surface, the segments being insulated from each other. Using such a segmented structure, the sensing and stimulating functions can be performed by separate segments. Further, the electrode surface used for sensing and stimulating can be varied during use, e.g. to increase the available electrode surface over the lifetime of the electrode or e.g. to increase stimulation on demand.

A pacemaker lead according to the invention comprises a flexible, insulated electrical lead extending between a connector portion and a tip portion, the tip portion being provided with a anchor electrode to be retained in the myocard of the heart, the anchor tip being at least partially covered with immunosuppressive of cytostatic agent.

The connector portion of the pacemaker lead is preferably embodied as a standard connector, e.g. a VS 1 international standard type connector.

The flexible, insulated wire may e.g. be a single or a bundle of helically wound electrically conducting wires disposed in a tubular structure of flexible material. When a plurality of wires is used, some of the wires may be insulated from other wires, e.g. to connect separate segments of the electrode.

A pacemaker according to the invention comprises a pulse generator for generating electrical impulses, the pulse generator being connected on a output side to at least one pacemaker lead, comprising a flexible, insulated electrical lead extending between a connector portion and a tip portion, the tip portion being provided with an anchor electrode to be retained in the myocard of the heart, the anchor electrode being at least partially covered with an immunosuppressive of cytostatic agent.

In accordance with the invention, an immunosuppressive agent is defined as any pharmaceutically acceptable, biologically active agent which is capable of at least partially inhibiting one or more reactions of an organism's immune system. Preferred examples of immunosuppressive agents for use in a coating for a lead tip according to the invention are cyclosporins, sirolimus (also known as rapamycin), tacrolimus, everolimus, FK-506, SDZ-RAD, and analogues of these agents having essentially the same pharmacological profile, in particular those analogues that are congeners that find the FKBP-12 protein. A particular preferred immunosuppressive agent is sirolimus, which compound is described in more detail in US patent 3,929,992, of which the contents are incorporated herein by reference.

A cytostatic agent is in accordance with the invention defined as any pharmaceutically acceptable, biologically active agent which reduces the rate of growth (proliferation) and division of cells. Preferred cytostatic agents for use in a coating for a lead tip according to the invention are oncostatic agents, in particular paclitaxel, taxotere, and other taxanes. Particularly preferred is paclitaxel.

The coating will generally contain from about 0.01 to about 10 mg, preferably from about 0.1 to about 4 mg of the immunosuppressive or cytostatic agent per cm² of the surface area of the lead tip.

Although it is possible to directly apply the immunosuppressive or cytostatic agent to the surface of the lead tip, it is preferred that it is incorporated into a polymeric matrix in which case the coating comprises both the immunosuppressive or cytostatic agent and the polymeric matrix. In accordance with this embodiment, the immunosuppressive or cytostatic agent will be released from the polymeric matrix in a preferably controlled manner. Preferably, the immunosuppressive or cytostatic agent is released with zero order kinetics over a period of at least three days up to about six months, more preferably between seven and thirty days.

The polymeric matrix may be of any biocompatible material which shows sufficient adherence to the material of the lead tip and is capable of releasing the immunosuppressive or cytostatic agent over time. Preferably, the polymeric matrix is of a biodegradable material wherein the rate of its degradation *in vivo* is proportional with the rate of release of the immunosuppressive or cytostatic agent. Of course, the material should be chosen such that its degradation products are also biocompatible and not toxic.

Suitable polymeric matrices are described for instance in US patent application 2002/0051730 for preparing coatings for stents. These coatings, when comprising a immunosuppressive or cytostatic agent, may, *mutatis mutandis,* also be applied to a lead tip according to the invention. Thus, the contents of said document are incorporated herein by reference. A non-limiting list of suitable polymeric materials include poly(ethylene-co-vinylacetate), polybutylmethacryate, polylactide (PLA), polyglycolide (PGA), copolymers of lactide and glycolide (PGLA), copolymers of lactide and caprolactone, and polyfluoro copolymers of monomers such as hexafluoropropylene (HFP), tetrafluoroethyelene (TFE), vinylidenefluoride, 1-hydropentafluoropropyelene, perfluoro(methyl vinyl ether), chlorotrifluoroethylene (CTFE), pentafluoropropene, trifluoroethylene, hexafluoroacetone, and/or hexafluoroisobutylene.

The lead tip of an electrode according to the invention having any shape or dimensions may be prepared using a sintering method. In accordance with this embodiment, the tip will be provided with small holes, pores or cavities wherein the immunosuppressive or cytotoxic agent may be contained. Advantageously, in accordance with this embodiment, the immunosuppressive or cytotoxic agent may be released from its position in a hole, pore or cavity when in position in the cardiac muscle.

In accordance with another embodiment, one or more concentric grooves may be provided around the tip of the electrode. The immunosuppressive or cytotoxic agent used in accordance with the invention may be contained in these grooves, to be released when the electrode is in use in the cardiac muscle.

The invention will now be illustrated further by the following, non-restrictive examples.

### Example 1

To establish an effect of rapamycin and paclitaxel on fibroblast growth, growth curves were made using a DNA measurement. Human fibroblasts were trypsinized and seeded in 24 well plates in a concentration of 20,000 cells per well. Different concentrations of rapamycin (obtained from Rapamune oral solution, produced by Wyeth Pharmaceuticals Inc., and obtained as a prescription drug from a common pharmacy) and paclitaxol (obtained from a common pharmacy as a prescription drug) were added 24 hours after seeding.

After 2 and 4 days, the plates were washed twice with saline and stored at -20°C until further analysis. The cells were extracted with ammonia solution (1 mmol/L) - Triton X100 (0.2% v/v) by sonification during 5 seconds at amplitude 15 (Soniprep 150; pH 7.0) was added. The remaining solution was mixed with Hoechst dye H33258 (100 µg/L).

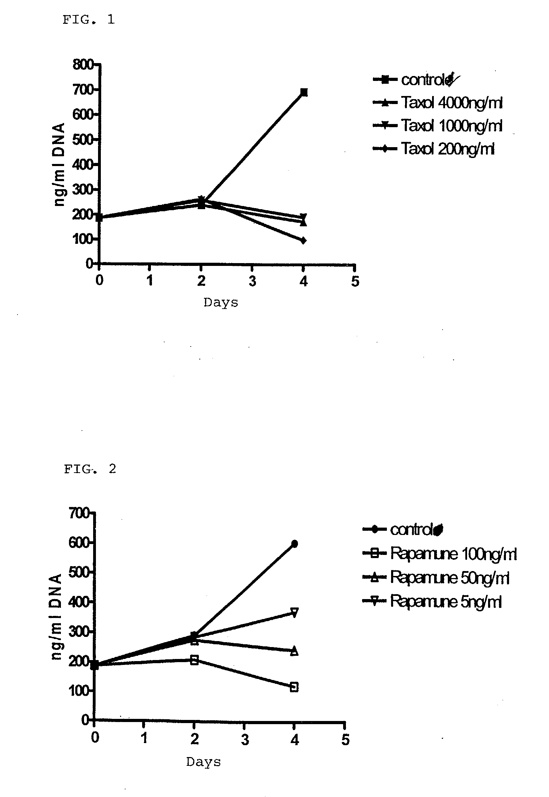

Fluorescence was measured after 10 minutes with the excitation and emission wavelengths set at 350 and 455 nm, respectively. The fluorescence of experimental samples was referenced to a standard curve of calf thymus DNA (type II, no D-3636; Sigma, Zwijndrecht, the Netherlands). The resulting growth curves are shown in Figures 1 and 2). These curves show in detail the growth of fibroblast cells in culture in the presence of different concentrations of paclitaxel (Figure 1) and rapamycin (Figure 2).

The invention will now be further elucidated on the basis of two exemplary embodiments that are represented in a drawing. In the drawing:
Fig. 3 shows a schematic perspective view of a pacemaker;
Fig. 4 shows a schematic perspective view of the lead tip of the pacemaker of fig. 1;
Fig. 5 shows a schematic perspective view of an alternative embodiment for the lead tip of fig. 2.

It is noted that the figures only concern schematic representations of preferred embodiments of the invention and that the figures are given as non-limiting examples. In the drawings, the same or corresponding parts are indicated by the same reference numerals.

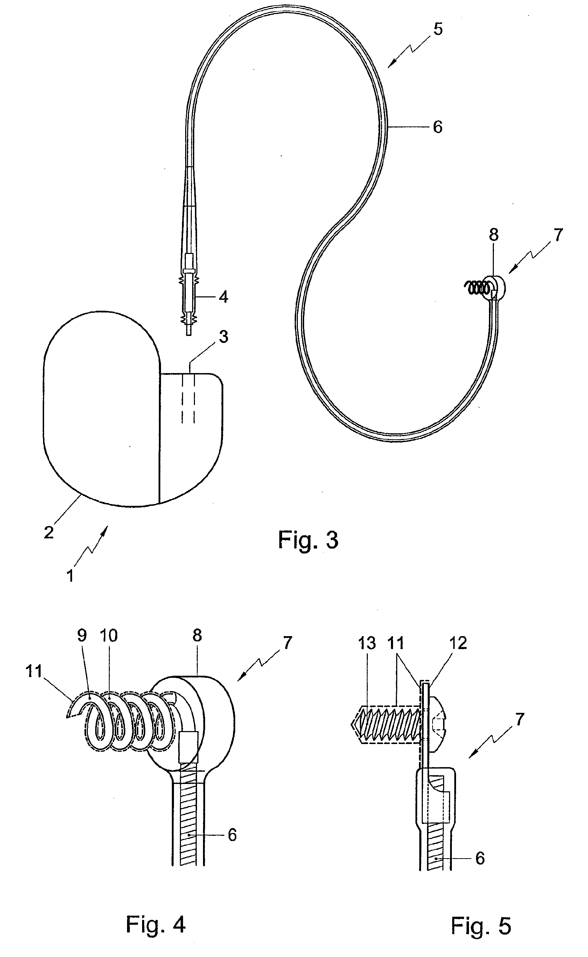

Fig. 3 shows a pacemaker 1, comprising a pulse generator 2 for generating electrical impulses. The pulse generator 2 is on an output side 3 of the pacemaker connected to a connector portion 4 of a pacemaker lead 5. In the drawing, the connector 4 is shown to be an EVS1 international standard type connector.

The pacemaker lead 5 comprises a flexible, insulated electrical lead 6 extending between the connector portion 4 and a tip portion 6. The electrical lead 6 comprises in this embodiment a bundle of helically wound electrically conducting wires that are disposed in a tubular sheath of silicone material.

Referring to fig. 4, the tip portion 7 of the pacemaker lead 5 comprises an anchor electrode 8 having a screw portion. In this embodiment, the screw portion is formed by a helically wound electrically conducting wire 9, having a sharpened tip.

The construction of the pulse generator, the pacemaker lead and the anchor electrode per se are well known to the skilled man and shall not be further elucidated.

The anchoring surface 10 of the electrode, in this embodiment the surface of the helically wound wire electrode 9 that in use cooperates with the myocard is coated with a coating 11 comprising the above-discussed immunosuppressive or cytostatic agent.

In fig. 5 an alternative embodiment is shown, is which the anchor electrode comprises a contacting plate portion 12 and a fastener portion 13. In this embodiment, the fastener portion 13 is formed as a screw of which the threaded portion forms the anchoring surface 10 that is coated with the immunosuppressive or cytostatic coating 11. In this embodiment, the contacting plate portion 12 may also be at least partially covered with the immunosuppressive or cytostatic agent.

As an alternative, the fastener may be formed as a staple.

These and other variations are considered to fall within the scope of the invention as set forth in the appended claims.

## Claims

1. Anchor electrode for a pacemaker lead, the anchor electrode having an anchoring surface being at least partially covered with an immunosuppressive of cytostatic agent.

2. Pacemaker lead, comprising a flexible, insulated electrical lead extending between a connector portion and a tip portion, the tip portion being provided with an anchor electrode to be retained in the myocard of the heart, the anchor electrode having an anchoring surface that is at least partially covered with an immunosuppressive of cytostatic agent.

3. Pacemaker, comprising a pulse generator for generating electrical impulses, the pulse generator being connected to at least one pacemaker lead, comprising a flexible, insulated electrical lead extending between a connector portion and tip portion, the tip portion being provided with an anchor electrode to be retained in the myocard of the heart, the anchor electrode having an anchoring surface that is at least partially covered with an immunosuppressive of cytostatic agent.

4. Use of an immunosuppressive or cytostatic agent as a coating material for coating the contacting surface of an anchor electrode for a pacemaker lead.

5. Use of an immunosuppressive or cytostatic agent in a coating for an anchor electrode for a pacemaker lead for reduction and/or prevention of scar tissue formation in the myocard of a human or animal heart to which the anchor electrode is attached.
